(19) Europäisches Patentamt
European Patent Office
Office européen des brevets

(11) Veröffentlichungsnummer: **0 035 957**
**B1**

# (12) EUROPÄISCHE PATENTSCHRIFT

(45) Veröffentlichungstag der Patentschrift: **27.07.83**

(51) Int. Cl.³: **C 07 D 231/24,** C 09 B 62/507

(21) Anmeldenummer: **81710009.2**

(22) Anmeldetag: **27.02.81**

(54) **Verfahren zur Herstellung von Schwefelsäurehalbester-Verbindungen.**

(30) Priorität: **11.03.80 DE 3009177**

(43) Veröffentlichungstag der Anmeldung: **16.09.81 Patentblatt 81/37**

(45) Bekanntmachung des Hinweises auf die Patenterteilung: **27.07.83 Patentblatt 83/30**

(84) Benannte Vertragsstaaten: **CH DE FR GB IT LI**

(56) Entgegenhaltungen:
**DE-A-2 431 343**
**DE-A-2 634 783**
**DE-A-2 634 856**
**DE-A-2 634 857**
**DE-B-1 215 282**

(73) Patentinhaber: **HOECHST AKTIENGESELLSCHAFT, Postfach 80 03 20, D-6230 Frankfurt/Main 80 (DE)**

(72) Erfinder: **Kohlhaas, Folker, Dr., Akazienring 64, D-6203 Hochheim am Main (DE)**
Erfinder: **Meininger, Fritz, Dr., Loreleistrasse 7, D-6230 Frankfurt am Main 80 (DE)**
Erfinder: **Hoyer, Ernst, Dr., Eptingweg 3, D-6230 Frankfurt am Main 80 (DE)**

**EP 0 035 957 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Schwefelsäurehalbester-Verbindungen

Die vorliegende Erfindung liegt auf dem Gebiet der Herstellung von Zwischenprodukten für faserreaktive Verbindungen. Sie betrifft die Herstellung von 1-($\beta$-Sulfatoäthylsulfonylphenyl)-pyrazolon(5)-Verbindungen durch Veresterung von 1-($\beta$-Hydroxyäthylsulfonyl-phenyl)-pyrazolon(5)-Verbindungen mittels eines Sulfatisierungsmittels in einem Lösungsmittel.

Die Veresterung von solchen Verbindungen ist an sich bekannt. Die bisher beschriebenen Methoden erfordern jedoch große Überschüsse an Schwefelsäure; diese überschüssige Schwefelsäure muß entweder bei der Aufarbeitung des Veresterungsproduktes und dessen Isolierung oder bei der Weiterverarbeitung des Veresterungsproduktes, z. B. zu Azofarbstoffen, mit Wasser verdünnt und neutralisiert werden und von dem Veresterungsprodukt oder dem Weiterverarbeitungsprodukt, wie dem Farbstoff, abgetrennt werden. Eine Rückgewinnung der Schwefelsäure ist somit praktisch ausgeschlossen. Die Säure belastet zudem als solche oder in neutralisierter Form als lösliches Sulfat das Abwasser.

Des weiteren haben die bekannten Veresterungsverfahren den Nachteil, daß die Veresterungsprodukte als Lösungen in Schwefelsäure anfallen. Diese Lösungen sind aber für eine Lagerung, beispielsweise für eine später erfolgende Weiterverarbeitung, wenig geeignet, so daß sie sogleich der Weiterverarbeitung, zum Beispiel der Herstellung für Farbstoffe, zugeführt werden müssen.

Ein Veresterungsverfahren obiger Art ist beispielsweise aus der deutschen Offenlegungsschrift 1 804 524 bekannt; so wird in den Beispielen 2 und 5 eine 1-($\beta$-Hydroxyäthylsulfonyl-phenyl)-5-pyrazolon-Verbindung mit der 7fach molaren Menge an konzentrierter Schwefelsäure umgesetzt. Nach dem Verdünnen der Lösung des Veresterungsproduktes in der Schwefelsäure mit viel Eis und Wasser und Neutralisation dieses großen Säureüberschusses wird die erhaltene Esterverbindung mit einer Diazoniumverbindung zu einem Azofarbstoff gekuppelt, der anschließend mit Natriumchlorid oder Kaliumchlorid ausgesalzen und auf diese Weise von dem hohen Sulfatballast befreit wird. Das bei der anschließenden Filtration in der Mutterlauge verbleibende Natriumsulfat sowie das zum Aussalzen zusätzlich in diese Lösung eingebrachte Natriumchlorid bzw. Kaliumchlorid bedeutet aber für das Abwasser eine erhebliche Salzbelastung.

In ähnlicher Weise erfolgt die Veresterung in den Beispielen 1 und 2 der deutschen Auslegeschrift 1 215 282 und im Beispiel 1 der deutschen Offenlegungsschrift 2 009 421, wobei jedoch noch höhere Überschüsse an Schwefelsäure eingesetzt werden. Im Beispiel 1 dieser DOS 2 009 421 verfährt man dann zur Herstellung des Farbstoffes in der Weise weiter, daß man die neutralisierte Lösung des Veresterungsproduktes mit einer Diazokomponente umsetzt und die so erhaltene Farbstofflösung mitsamt dem Natriumsulfat aus dem 13fach molaren Schwefelsäureüberschuß der Sprühtrocknung unterwirft. Das auf diese Weise erhältliche Farbstoffpulver fällt jedoch sehr farbschwach und mit einem hohen Prozentgehalt an Neutralsalzen an, so daß es nur von geringem technischen Interesse sein düfte.

Eine Neutralisation der überschüssigen Schwefelsäure mit Calciumcarbonat und Abfiltration des schwer löslichen Calciumsulfats, wie im Beispiel 1 der deutschen Offenlegungsschrift 2 431 343 beschrieben, entlastet zwar das Abwasser, erfordert aber einen zusätzlichen Arbeits- und Materialaufwand; des weiteren hat diese Methode den Nachteil, daß das so anfallende Calciumsulfat als unverwertbarer Industriemüll deponiert werden muß.

Es ist weiterhin aus der deutschen Offenlegungsschrift 1 443 877 von Beispiel 1 bekannt, daß man eine 1-($\beta$-Hydroxyäthylsulfonyl-phenyl)-pyrazolon(5)-Verbindung mittels Amidosulfonsäure in Pyridin als Lösungsmittel in die entsprechende Schwefelsäurehalbesterverbindung überführen kann. Verwendet wird die Amidosulfonsäure als Sulfatisierungsmittel in der 3,6fach äquimolaren Menge. Dies bedeutet zwar eine deutliche Verminderung des Überschusses an Veresterungsmittel, jedoch muß in der Folge das eingesetzte Pyridin unter vermindertem Druck weitgehend abdestilliert werden. Trotzdem gelangt dabei noch etwa ein Viertel dieses intensiv und unangenehm riechenden, wasserlöslichen Pyridins bei der Weiterverarbeitung des Schwefelsäurehalbesters zu dem Azofarbstoff in die Mutterlauge, aus der es entfernt werden muß, damit es nicht in die Abwässer gelangt.

Ein weiteres, jedoch verfahrenstechnisch anders geführtes und somit zu den vorstehend besprochenen Verfahren nicht vergleichbares Verfahren zur Sulfatierung von $\beta$-hydroxyäthylsulfonylgruppenhaltigen Pyrazolonverbindungen ist aus der deutschen Offenlegungsschrift 2 634 783 bekannt.

Dieses Verfahren hat zwar den Vorteil, mit sehr geringen Mengen an Sulfatierungsmittel auszukommen, muß jedoch in Knetmaschinen durchgeführt werden.

Es bestand deshalb ein dringender Bedarf nach einem Veresterungsverfahren zur Herstellung solcher $\beta$-Sulfatoäthylsulfonylphenyl-pyrazolon-Verbindungen, das die Nachteile der Veresterung in Schwefelsäure als Lösungsmittel als auch der Veresterung in Pyridin als Lösungsmittel vermeidet, jedoch die Vorteile des Arbeitens in einem organischen Lösungsmittel und insbesondere vorteilhaft den Einsatz geringer Mengen Veresterungsmittel bietet. Mit der vorliegenden Erfindung wurde solch eine Aufgabe gelöst.

Die vorliegende Erfindung betrifft ein Verfahren zur Herstellung von Verbindungen der allgemeinen

Formel (1)

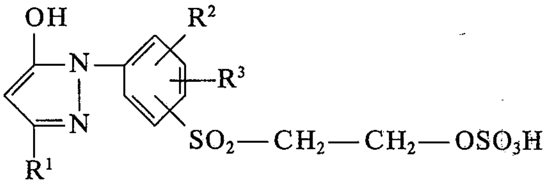

(1)

in welcher $R^1$ für eine Methylgruppe, für die Carboxygruppe oder für die Phenylgruppe, die durch 1 oder 2 Substituenten aus der Gruppe Methyl, Äthyl, Methoxy, Äthoxy und Chlor substituiert sein kann, steht, $R^2$ ein Wasserstoffatom, eine Methyl- oder Äthylgruppe, eine Methoxy- oder Äthoxygruppe, oder ein Chloratom bedeutet, $R^3$ ein Wasserstoffatom, eine Methyl- oder Äthylgruppe, oder eine Methoxy- oder Äthoxygruppe, darstellt und die $\beta$-Sulfatoäthylsulfonyl-Gruppe in 3'- oder 4'-Stellung des Phenylrestes gebunden ist,
durch Sulfatierung einer Verbindung der allgemeinen Formel (2)

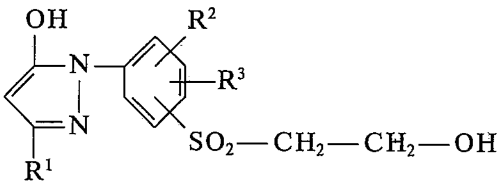

(2)

in welcher $R^1$, $R^2$ und $R^3$ die obengenannten Bedeutungen haben und die $\beta$-Hydroxyäthylsulfonyl-Gruppe in 3'- oder 4'-Stellung des Phenylrestes gebunden ist, in einem organischen Lösungsmittel, das dadurch gekennzeichnet ist, daß die Veresterung in N-Methyl-pyrrolidon als Lösungsmittel und mit Schwefeltrioxid oder Chlorsulfonsäure als Sulfatierungsmittel durchgeführt wird.

Die Verwendung von N-Methyl-pyrrolidon als Lösungsmittel hat den großen Vorteil, daß es bei Aufarbeitung des Reaktionsansatzes aus der erhältlichen wäßrigen Phase leicht extrahiert und später leicht destillativ abgetrennt werden kann.

Das Schwefeltrioxid kann als Substanz oder in N-Methylpyrrolidon gelöst, ebenso aber auch in konzentrierter Schwefelsäure gelöst, beispielsweise als 30 bis 70gew.-%iges Oleum, in den Reaktionsansatz eingebracht werden.

Die erfindungsgemäße Verfahrensweise, in N-Methylpyrrolidon und mit Schwefeltrioxid oder Chlorsulfonsäure zu arbeiten, hat den Vorteil, daß diese Veresterungsmittel nur in sehr geringen Mengen eingesetzt zu werden brauchen. So kann man diese Sulfatierungsmittel schon in der 2fach molaren Menge, bezogen auf die Ausgangsverbindung der allgemeinen Formel (2), einsetzen. Das Sulfatierungsmittel kann vorteilhaft in der 1- bis 2fach molaren Menge, bezogen auf die Ausgangsverbindung der allgemeinen Formel (2), eingesetzt werden. Ganz besonders vorteilhaft ist die Verwendung des Veresterungsmittels zur Ausgangsverbindung der allgemeinen Formel (2) im Molverhältnis von 1 : 1 bis 1,6 : 1. Auch bei diesen geringen Mengen an Veresterungsmittel werden Ausbeuten von über 98% d. Th. erhalten.

Das Arbeiten mit den geringen Mengen an Veresterungsmitteln hat weiterhin den Vorteil, daß bei der Aufarbeitung durch Neutralisation nur eine geringe Menge an Neutralisierungsmittel und somit nur eine geringe Menge an Salzen, wie Sulfaten oder Chloriden, die in den bisher bekannten Verfahren eine starke Belastung für das Abwasser darstellen, auftreten.

Die erfindungsgemäße Verfahrensweise der Veresterung kann sowohl bei niedrigen als auch bei hohen Temperaturen durchgeführt werden. So kann die Umsetzung bei Temperaturen zwischen 0°C und 120°C erfolgen; vorzugsweise arbeitet man bei einer Temperatur zwischen 10 und 70°C.

Nach Beendigung der Reaktion kann das entstandene homogene Reaktionsgemisch wie folgt aufgearbeitet werden: Man verdünnt es mit Wasser, bzw. unter Kühlen oder mit Eiswasser, und gibt zur Neutralisation der Säure ein übliches säurebindendes Mittel, wie ein basisch reagierendes Alkalimetallsalz, beispielsweise Natriumcarbonat oder Natriumbicarbonat, bis zu einem pH-Wert von 4,5 bis 7 hinzu. Sodann wird diese wäßrige Lösung mit einem hierfür geeigneten organischen Lösungsmittel, wie beispielsweise Methylenchlorid, Chloroform oder 1,2-Dichloräthan, extrahiert.

Die Verbindungen der allgemeinen Formel (1) liegen so dann in der wäßrigen Phase gelöst als neutrale Salze vor; in diesen wäßrigen Lösungen befinden sich, wie schon erwähnt, bei der Verwendung der geringen Mengen an dem Veresterungsmittel nur geringe Mengen an Elektrolyten, so bei der Verwendung von Schwefeltrioxid nur geringe Mengen an den Alkalimetallsulfaten und bei Verwendung von Chlorsulfonsäure nur geringe Mengen an Alkalimetallchloriden, so daß diese Verbindungen der allgemeinen Formel (2), in Form der Salze, leicht durch Eindampfen der wäßrigen Phase, beispielsweise durch Sprühtrocknung, isoliert werden können. Sie fallen auf diese Weise sehr salzarm an, und das »Abwasser« enthält keine Substanzen.

Die organische Phase, die bei der Extraktion gewonnen wird und die die organischen,

extrahierenden Lösungsmittel und das N-Methylpyrrolidon enthält, läßt sich anschließend leicht destillativ aufarbeiten, so daß diese Lösungsmittel praktisch verlustfrei in den Reaktions- und Aufarbeitungsprozeß zurückgeführt werden können.

Ein weiterer Vorteil des erfindungsgemäßen Verfahrens liegt darin, daß die erhältliche wäßrige Phase mit den Salzen der Verbindungen der allgemeinen Formel (1), die nach der Extraktion des N-Methylpyrrolidons erhalten wird, auch direkt zu weiteren Umsetzungen eingesetzt werden kann, ohne daß diese Verbindungen zuvor isoliert werden müssen. Die Verbindungen der allgemeinen Formel (1) stellen bekannte Kupplungskomponenten dar, die bspw. in üblicher und bekannter Weise mit Diazokomponenten in Azofarbstoffe übergeführt werden können. Wegen des geringen Anteils an Elektrolyten kann man die wäßrige Phase mit diesen Verbindungen der allgemeinen Formel (1) direkt in den Kupplungsprozeß einsetzen, und man erhält so Syntheselösungen von faserreaktiven Azofarbstoffen mit einem ebenfalls geringen Elektrolytgehalt. Das bedeutet, daß auch für die Weiterverarbeitung der erfindungsgemäß hergestellten Verbindungen eine umweltfreundliche, abwasserfreie Synthese der Azofarbstoffe möglich wird und bei Verwendung der wäßrigen Phase mit den Verbindungen der allgemeinen Formel (1) selbst eine vorherige Abscheidung oder Isolierung dieser Kupplungskomponenten nicht mehr erforderlich ist. Die auf diese Weise leicht mit den Kupplungskomponenten der allgemeinen Formel (1) erhältlichen Azofarbstoffe oder auch deren Metallkomplexverbindungen, insbesondere Kupfer-, Kobalt- und Chromkomplexverbindungen, sofern die Diazokomponente o-ständig zur Amino- bzw. Azogruppe eine Hydroxy- oder Carboxygruppe enthält und man ein metallabgebendes Mittel hinzugibt, können deshalb ebenso aus den Syntheselösungen durch Eindampfen, beispielsweise Sprühtrocknung, als elektrolytarme Farbstoffpulver isoliert werden, und die Abscheidung der Farbstoffe durch Aussalzen mit Natriumchlorid oder Kaliumchlorid ist nicht mehr erforderlich. Man erhält deshalb bei Einsatz der erfindungsgemäß hergestellten Verbindungen der allgemeinen Formel (1) farbstarke Farbstoffpulver in sehr guter Ausbeute bei ausgezeichneter Qualität und Reinheit, die in ihren Eigenschaften gegenüber den Farbstoffprodukten, die mit entsprechenden Ausgangsverbindungen aus bisher üblichen Veresterungsverfahren erhalten wurden, Vorteile bieten, insbesondere wegen des verbesserten Veresterungsgrades der β-Hydroxyäthylsulfonylgruppe eine höhere Farbstärke, darüber hinaus eine höhere Löslichkeit in Wasser und eine bessere Farbstoffausbeute aufweisen. Bei genügend großer Löslichkeit dieser Farbstoffe, die unter Einsatz der erfindungsgemäß hergestellten Verbindungen der allgemeinen Formel (1) synthetisiert wurden, können die aus dieser Synthese anfallenden Farbstofflösungen auch direkt oder nach Einengen auf ein geringeres Volumen für färberische Zwecke eingesetzt werden. Auch dieser Vorteil ist eine Folge des erfindungsgemäßen Verfahrens zur Herstellung der Verbindungen der allgemeinen Formel (1).

Die nachstehenden Beispiele dienen zur Erläuterung der Erfindung. Die Teile sind Gewichtsteile, die Prozentangaben beziehen sich auf Gewichtsprozente, sofern nicht anders vermerkt. Gewichtsteile verhalten sich zu Volumenteilen wie Kilogramm zu Liter.

## Beispiel 1

84,6 Teile 1-[4'-(β-hydroxyäthylsulfonyl)-phenyl]-3-methylpyrazolon(5) werden in 180 Volumenteilen N-Methylpyrrolidon eingetragen. Bei einer Temperatur von etwa 20°C werden innerhalb von etwa 30 Minuten unter Rühren 26 Volumenteile Chlorsulfonsäure zugetropft. Nach Beendigung der Reaktion (Reaktionszeit etwa 60 Minuten) wird das Reaktionsgemisch mit 800 Teilen Eiswasser verdünnt und mit 75 Teilen Natriumbicarbonat auf einen pH-Wert von 5,5 bis 6 eingestellt. Zur Abtrennung des N-Methylpyrrolidons wird die erhaltene Lösung mit 500 Volumenteilen Methylenchlorid extrahiert. Nach Trennung der Phasen werden etwa 650 Volumenteile einer wäßrigen Lösung erhalten, die 123 Teile des Natriumsalzes entsprechend der Verbindung der Formel

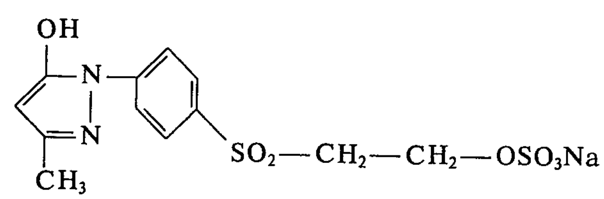

enthält, welche durch Eindampfen der wäßrigen Lösung, beispielsweise durch Sprühtrocknung isoliert werden kann. Der Elektrolytgehalt beträgt 16%. Die Ausbeute beträgt 96% d. Th. mit einem Veresterungsgrad von 99,2%.

Beispiel 2

Zu einer Lösung von 31 Teilen Schwefeltrioxid in 180 Vol.-Teilen N-Methylpyrrolidon werden bei etwa 22°C innerhalb von etwa 30 Minuten 84,6 Teile 1-[4'-(β-Hydroxyäthylsulfonyl)-phenyl]-3-methyl-pyrazolon(5) unter Rühren eingetragen. Nach Beendigung der Reaktion (Reaktionszeit etwa 60 Minuten) wird das Reaktionsgemisch gemäß den Angaben des Beispieles 1 aufgearbeitet. Die erhaltene wäßrige Lösung wird eingedampft. Es werden 137 Teile eines Pulvers erhalten, das 90,4% des in Beispiel 1 formelmäßig angegebenen Natriumsalzes enthält. Der Elektrolytgehalt beträgt 9%, der Veresterungsgrad 99,5%. Die Ausbeute beträgt demnach 97% d. Th.

Beispiel 3

Man verfährt wie im Beispiel 2 beschrieben, setzt jedoch anstelle von 31 Teilen Schwefeltrioxid 48 Teile 65%iges Oleum ein.

Man erhält 161 Teile eines Produktes, das 77% des in Beispiel 1 formelmäßig angegebenen Natriumsalzes bei einem Elektrolytgehalt von 23% enthält. Der Veresterungsgrad beträgt 99,6%, die Ausbeute 97% d. Th.

Beispiele 4 bis 22

Verfährt man in erfindungsgemäßer Weise zur Herstellung der Esterverbindungen der allgemeinen Formel (1), beispielsweise in analoger Weise zu den Verfahrensvarianten, die in den Beispielen 1, 2 oder 3 dort für eine Verbindung beschrieben sind, und geht hierbei von anderen Ausgangsverbindungen entsprechend der allgemeinen Formel (2) aus, wie sie beispielsweise in den nachfolgenden Tabellenbeispielen aufgeführt sind, so erhält man entsprechende Veresterungsprodukte mit einem Veresterungsgrad von über 98% und Ausbeuten von über 95% d. Th.

| Bsp. | Verbindungen der allgemeinen Formel (1) bzw. der allgemeinen Formel (2) | | | |
|---|---|---|---|---|
| | $R^1$ | $R^2$ | $R^3$ | β-Hydroxy- bzw. β-Sulfatoäthylsulfonyl in ...-Stellg. |
| 4 | $CH_3$ | 2'-$CH_3$ | H | 3'– |
| 5 | COOH | H | H | 4'– |
| 6 | COOH | H | H | 3'– |
| 7 | Phenyl | H | H | 4'– |
| 8 | Phenyl | H | H | 3'– |
| 9 | $CH_3$ | H | H | 3'– |
| 10 | $CH_3$ | 2'-$OCH_3$ | H | 3'– |
| 11 | $CH_3$ | 2'-$OCH_3$ | 3'-$CH_3$ | 4'– |
| 12 | $CH_3$ | 2'-$OCH_3$ | 3'-$OCH_3$ | 4'– |
| 13 | $CH_3$ | 2'-Cl | H | 4'– |
| 14 | COOH | 2'-$CH_3$ | H | 3'– |
| 15 | COOH | 2'-$OCH_3$ | H | 3'– |
| 16 | COOH | 2'-$OCH_3$ | 3'-$CH_3$ | 4'– |
| 17 | COOH | 2'-$OCH_3$ | 3'-$OCH_3$ | 4'– |
| 18 | Phenyl | 2'-$CH_3$ | H | 3'– |
| 19 | Phenyl | 2'-$OCH_3$ | H | 3'– |
| 20 | Phenyl | 2'-$OCH_3$ | 3'-$CH_3$ | 4'– |
| 21 | Phenyl | 2'-$OCH_3$ | 3'-$OCH_3$ | 4'– |
| 22 | Phenyl | 2'-$OCH_3$ | 3'-$OCH_3$ | 4'– |

Beispiel 23 (Anwendung)

75,8 Teile 2-Aminonaphthalin-1,5-disulfonsäure werden in einer Mischung aus 300 Teilen Wasser, 300 Teilen Eis und 75 Volumenteilen einer 31%igen Salzsäure bei 0 bis 10° C mit 50 Volumenteilen einer wäßrigen 5n-Natriumnitritlösung diazotiert. 105,8 Teile des gemäß Beispiel 2 hergestellten Natriumsalzes des 1-[4'-(β-Sulfatoäthylsulfonyl)-phenyl]-3-methyl-pyrazolon-5-ons werden als Substanz in 500 Teilen Wasser gelöst (besonders vorteilhaft kann man anstelle der hier hergestellten wäßrigen Lösung dieses Natriumsalzes direkt von der wäßrigen Lösung des nach der Aufarbeitung der Veresterungsreaktion erhältlichen wäßrigen Phase ausgehen, die dieses Natriumsalz bereits gelöst enthält). Diese wäßrige Lösung wird zu der oben hergestellten Suspension des Diazoniumsalzes gegeben. Es wird ein pH-Wert von 3,5 bis 4,0 mit 40 Teilen wasserfreiem Natriumcarbonat eingestellt. Nach beendeter Kupplung wird der Reaktionsansatz langsam auf 60 bis 70° C erwärmt und die Lösung mit 10 Teilen Kieselgur geklärt. Das Filtrat wird mit wasserfreiem Natriumcarbonat auf einen pH-Wert von 4,8 bis 5,3 eingestellt und anschließend bei 50 bis 60° C zur Trockene eingedampft. Nach Mahlung des Rückstandes erhält man 284 Teile eines gelben Pulvers, das 60%ig an dem Farbstoff der Formel

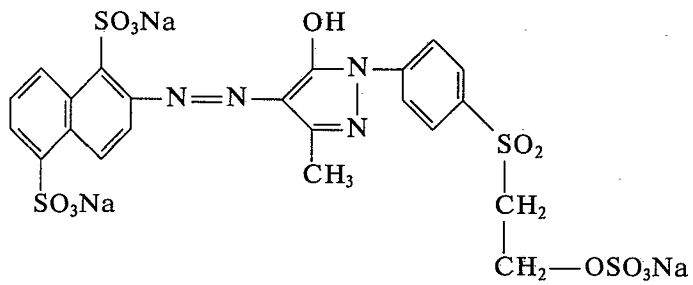

ist. Dieser Farbstoff liefert in Gegenwart alkalisch wirkender Mittel auf Baumwolle und anderen Cellulosefasermaterialien in den für Reaktivfarbstoffen bekannten üblichen Färbeverfahren farbstarke, klare, grünstichig gelbe Färbungen und Drucke von sehr guten Licht- und Naßechtheiten.

Beispiel 24 (Anwendung)

Man diazotiert 78,5 Teile 2-Aminonaphthalin-6,8-disulfonsäure in üblicher Weise und verwendet als Kupplungskomponente die gemäß Beispiel 1 nach Aufarbeitung des Reaktionsansatzes erhältliche wäßrige Lösung des Natriumsalzes des Veresterungspoduktes. 552 Volumenteile dieser wäßrigen Lösung werden mit der Diazoniumsalzsuspension in analoger Weise, wie im Beispiel 23 beschrieben, gekuppelt. Nach Aufarbeitung erhält man 279 Teile eines gelben Farbstoffpulvers mit einem Gehalt von 21% an dem Farbstoff der Formel

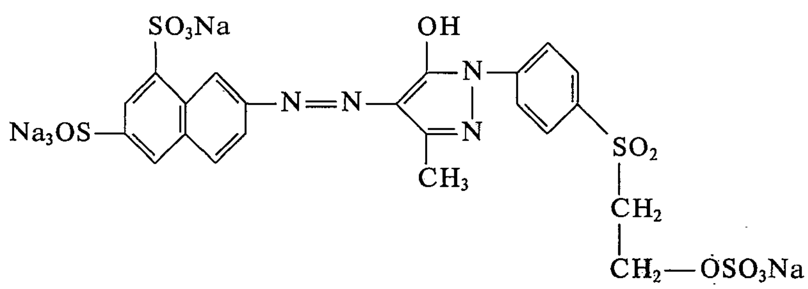

dieser Farbstoff liefert in Gegenwart alkalisch wirkender Mittel auf Baumwolle nach den für Reaktivfarbstoffe üblichen und bekannten Färbe- und Druckverfahren farbstarke, gelbe Färbungen und Drucke von sehr guten Licht- und Naßechtheiten.

Beispiel 25 (Anwendung)

37,8 Teile 2-Aminophenol-4-sulfonsäure werden in einer Mischung aus 150 Teilen Wasser, 150 Teilen Eis und 15 Teilen 100%iger Schwefelsäure mit einer wäßrigen Lösung von 14 Teilen Natriumnitrit diazotiert. Nach einstündigem Nachrühren wird ein geringer Nitritüberschuß durch Zugabe von wenig Amidosulfonsäure zerstört. In diese Diazoniumsalzsuspension werden 85 Teile des nach Beispiel 2 erhaltenen Natriumsalzes der veresterten Pyrazolonverbindung eingetragen; die Reaktionsmischung wird mit Natriumcarbonat auf einen pH-Wert von 6 bis 7 eingestellt und die Kupplung wird bei Raumtemperatur unter Rühren im Verlauf von 15 bis 20 Stunden zu Ende geführt. Anschließend werden diesem Reaktionsansatz 50 Teile kristallisiertes Kupfersulfat zugesetzt und der pH-Wert wird mit Natriumcarbonat auf 4,5 bis 5,5 eingestellt. Die Kupferung ist nach etwa einer Stunde bei Raumtemperatur beendet; die Farbstofflösung wird so dann mit 10 Teilen Kieselgur geklärt und das Filtrat wird sprühgetrocknet.

Es werden 215 Teile eines gelbbraunen Farbstoffpulvers erhalten, das 60%ig an dem Farbstoff der Formel

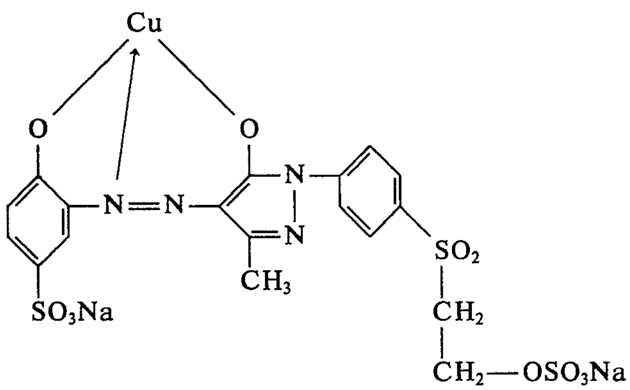

ist. Dieser Farbstoff liefert in Gegenwart alkalisch wirkender Mittel auf Baumwolle und anderen Cellulosefasermaterialien in den für Reaktivfarbstoffe bekannten und üblichen Färbeweisen farbstarke, gelbbraune Färbungen und Drucke von vorzüglichen Licht- und Naßechtheiten.

In üblicher und bekannter Weise, beisspielsweise wie in den Beispielen 23 bis 25 beschrieben, lassen sich andere Azofarbstoffe und Kupfer-, Chrom- und Kobaltkomplexfarbstoffe herstellen, wenn man von den erfindungsgemäß hergestellten Verbindungen der allgemeinen Formel (1) wie sie beispielsweise in den Beispielen 1 bis 22 beschrieben sind, als Kupplungskomponenten ausgeht. Die mit diesen Ausgangsverbindungen erhältlichen Farbstoffe besitzen ebenso sehr klare Nuancen und gute Echtheiten ihrer Färbungen auf Baumwolle oder Wolle.

**Patentansprüche**

1. Verfahren zur Herstellung von Verbindungen der allgemeinen Formel (1)

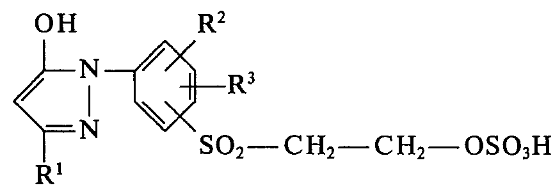

(1)

in welcher $R^1$ für eine Methylgruppe, für die Carboxygruppe oder für die Phenylgruppe, die durch 1 oder 2 Substituenten aus der Gruppe Methyl, Äthyl, Methoxy, Äthoxy und Chlor substituiert sein kann, steht $R^2$ ein Wasserstoffatom, eine Methyl- oder Äthylgruppe, eine Methoxy- oder Äthoxygruppe oder ein Chloratom bedeutet, $R^3$ ein Wasserstoffatom, eine Methyl- oder Äthylgruppe oder eine Methoxy- oder Äthoxygruppe darstellt und die $\beta$-Sulfatoäthylsulfonyl-Gruppe in 3'- oder 4'-Stellung des Phenylrestes gebunden ist,
durch Sulfatierung einer Verbindung der allgemeinen Formel (2)

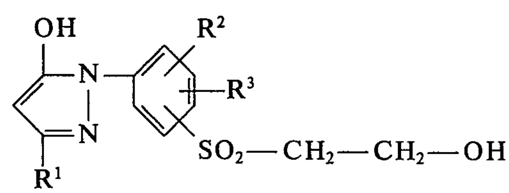

(2)

in welcher $R^1$, $R^2$ und $R^3$ die obengenannten Bedeutungen haben und die $\beta$-Hydroxyäthylsulfonyl-Gruppe in 3'- oder 4'-Stellung des Phenylrestes gebunden ist, in einem organischen Lösungsmittel, dadurch gekennzeichnet, daß die Veresterung in N-Methylpyrrolidon als Lösungsmittel und mit Schwefeltrioxid oder Chlorsulfonsäure als Sulfatierungsmittel durchgeführt wird.

2. Verfahren nach Anspruch 1, dadurch gekennzeichnet, daß das Sulfatierungsmittel in der 1- bis 2fach molaren Menge, bezogen auf die Ausgangsverbindung der allgemeinen Formel (2), eingesetzt wird.

3. Verfahren nach Anspruch 1 oder 2, dadurch gekennzeichnet, daß man die Sulfatierung bei einer Temperatur zwischen 10° und 70° C durchführt.

4. Verfahren nach Anspruch 1, 2 oder 3, dadurch gekennzeichnet, daß man den nach Beendigung der Reaktion erhältlichen Reaktionsansatz mit Wasser verdünnt und mit einem säurebindenden Mittel auf einen pH von 4,5 bis 7 einstellt und diese wäßrige Lösung mit dem Salz der Verbindung der allgemeinen Formel (1) durch Extraktion mit einem organischen Lösungsmittel von N-Methylpyrrolidon befreit.

5. Verwendung der nach Anspruch 4 erhältlichen wäßrigen Lösung (Phase) mit dem Salz der Verbindung der allgemeinen Formel (1) dadurch gekennzeichnet, daß man diese wäßrige Lösung direkt als Lösung einer Kupplungskomponente in die Synthese von Azofarbstoffen oder Metallkomplexazofarbstoffen einsetzt.

**Claims**

1. Process for the preparation of compounds of the general formula (1)

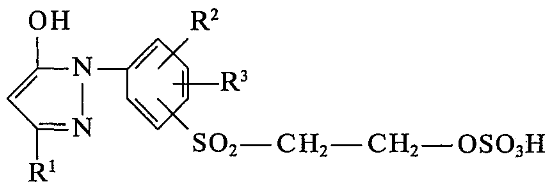

(1)

in which $R^1$ represents a methyl group, the carboxy group or the phenyl group which can be substituted by one or two substituents from the group methyl, ethyl, methoxy, ethoxy and chlorine, $R^2$ means a hydrogen atom, a methyl or ethyl group, a methoxy or ethoxy group or a chlorine atom, $R^3$ represents a hydrogen atom, a methyl or ethyl group or a methoxy or ethoxy group, and the $\beta$-sulfatoethylsulfonyl group is bonded in the 3'- or 4'-position of the phenyl radical,
by sulfating, in an organic solvent, a compound of the general formula (2)

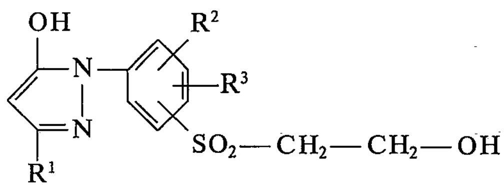

in which $R^1$, $R^2$ and $R^3$ have the above-mentioned meanings and the $\beta$-hydroxyethylsulfonyl group is bonded in the 3'- or 4'-position of the phenyl radical, characterized by that the esterification is carried out in N-methyl-pyrrolidone as a solvent and by means of sulfur trioxide or chlorosulfonic acid as a sulfating agent.

2. Process according to claim 1, characterized in that the sulfating agent is used in the 1- to 2-times molar amount calculated on the starting compound of the general formula (2).

3. Process according to claim 1 or 2, characterized in that the sulfatation is carried out at a temperature between 10 and 70° C.

4. Process according to claim 1, 2 or 3, characterized in that the reaction bath obtainable after the termination of the reaction is diluted with water and adjusted to a pH of from 4.5 to 7 with an acid-binding agent, and that this aqueous solution containing the salt of the compound of the general formula (1) is freed of N-methylpyrrolidone by extraction with an organic solvent.

5. Use of the aqueous solution (phase) containing the salt of the compound of the general formula (1), obtainable according to claim 4, characterized by that this aqueous solution is employed directly as solution of a coupling component in the synthesis of azo dyestuffs or metalcomplex azo dyestuffs.

**Revendications**

1. Procédé de préparation de composés répondant à la formule générale (1)

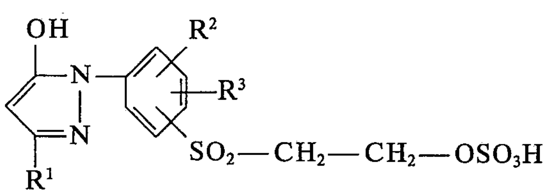

(1)

dans laquelle $R^1$ désigne un groupe méthyle, le groupe carboxy ou le groupe phényle, qui peut être substitué par 1 ou 2 substituants du groupe méthyle, éthyle, méthoxy, éthoxy et chlore, $R^2$ un atome d'hydrogène un groupe méthyle ou éthyle un groupe méthoxy ou éthoxy ou un atome de chlore, $R^3$ un atome d'hydrogène, un groupe méthyle ou éthyle ou un groupe méthoxy ou éthoxy et le groupe sulfatoéthylsulfonyle est lié en position 3' ou 4' du radical phényle,
par sulfatation d'un composé répondant à la formule générale (2)

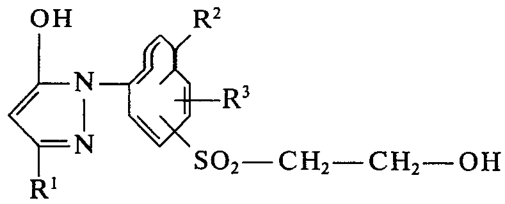

(2)

dans laquelle $R^1$, $R^2$ et $R^3$ ont les significations indiquées ci-dessus et le groupe $\beta$-hydroxyéthylsulfonyle est lié en position 3′ ou 4′ du radical phényle, dans un solvant organique, caractérisé en ce que l'estérification est effectuée dans la N-méthylpyrrolidone comme solvant et avec du trioxyde de soufre ou de l'acide chlorosulfonique comme agent de sulfatation.

2. Procédé suivant la revendication 1, caractérisé en ce que l'agent de sulfatation est utilisé dans une quantité 1 à 2 fois molaire, par rapport au composé de départ répondant à la formule générale (2).

3. Procédé suivant les revendications 1 ou 2, caractérisé en ce que la sulfatation est effectuée à une température comprise entre 10 et 70° C.

4. Procédé suivant les revendications 1, 2 ou 3, caractérisé en ce qu'on dilue avec de l'eau le mélange réactionnel pouvant être obtenu après la fin de la réaction et on l'ajuste à un pH de 4,5 à 7 avec un agent fixant les acides et on débarrasse cette solution aqueuse avec le sel du composé répendant à la formule générale (1), par extraction avec un solvant organique, de la N-méthylpyrrolidone.

5. Utilisation de la solution (phase) aqueuse avec le sel du composé répondant à la formule générale (1), caractérisée en ce qu'on utilise directement cette solution aqueuse comme solution d'un constituant de copulation dans la synthèse de colorants azoïques ou de colorants azoïques à complexes métalliques.